# EUROPEAN PATENT SPECIFICATION

(11) **EP 3 406 256 B1**
(45) Date of publication and mention of the grant of the patent: **18.11.2020**
(21) Application number: 17741609.6
(22) Date of filing: 11.01.2017
(51) Int. Cl.: A61K 36/185, A61K 36/25, A61K 9/08, A61K 47/36, A61P 11/00

(54) **COMPOSITE LIQUID PHARMACEUTICAL COMPOSITION COMPRISING CRUDE DRUG-STABILIZING DRIED POWDER OF HEDERA HELIX AND PELARGONIUM SIDOIDES**
PHARMAZEUTISCHE VERBUNDFLÜSSIGKEITSZUSAMMENSETZUNG MIT ROHEM WIRKSTOFFSTABILISIERENDEN GETROCKNETEN HEDERA HELIX UND PELARGONIUM SIDOIDES PULVER
COMPOSITION PHARMACEUTIQUE LIQUIDE COMPOSITE À BASE D'UNE POUDRE SÉCHÉE DE STABILISATION DE MÉDICAMENT BRUTE COMPRENANT HEDERA HELIX ET PELARGONIUM SIDOIDES

(30) Priority: 19.01.2016 KR 20160006463
(43) Date of publication of application: 28.11.2018
(73) Proprietor: Yang Ji Chemical Co., Ltd., Gyeonggi-do 16229 (KR); Han Wha Pharma Co., Ltd., Chuncheon-si, Gangwon-do 24468 (KR)
(72) Inventor: KIM, Kyoung Rak, Seoul 06600 (KR); MOON, Hong Sik, Suwon-si Gyeonggi-do 16505 (KR); KIM, Tae Wook, Seoul 06567 (KR); KIM, Sung Jun, Yongin-si Gyeonggi-do 16928 (KR); YU, Se Mi, Suwon-si Gyeonggi-do 16219 (KR); YOON, Yeong Cheol, Seongnam-si Gyeonggi-do 13597 (KR); KIM, Jong Hoon, Anyang-si Gyeonggi-do 14102 (KR)
(74) Representative: dompatent von Kreisler Selting Werner - Partnerschaft von Patent- und Rechtsanwälten mbB
(86) International application number: PCT/KR2017/000369
(87) International publication number: WO 2017/126843

(56) References cited:
- WO-A1-03/028746
- WO-A1-2014/060525
- KR-A- 20100 016 615
- KR-A- 20100 016 635
- KR-A- 20150 050 831

## Description

### TECHNICAL FIELD

The present invention relates to a liquid pharmaceutical composition including a stabilized dry powder of *Pelargonium sidoides,* and specifically, to a composite liquid pharmaceutical composition including a stabilized dry powder of *Pelargonium sidoides,* in which storage stability of a main component of *Pelargonium sidoides* is improved during preparation of the composite liquid formulation, and a preparation method of the liquid pharmaceutical composition.

### BACKGROUND ART

Respiratory diseases include respiratory infectious diseases such as the common cold, bronchitis, sinusitis, tonsillitis, sore throat, etc., asthma, bronchitis, chronic obstructive pulmonary disease, etc. These respiratory diseases are known to be attributed to inflammation caused by respiratory infections or immune responses. Although respiratory diseases vary in degree depending on specific diseases, they all cause a deterioration in quality of life. In severe cases, respiratory diseases render patients dependent on other people for their daily activities, leading to deterioration in productivity and quality of life for those around the patients.

From bronchial stenosis, the concept of asthma has been reestablished as a chronic bronchial inflammatory disease. When symptoms are present, it is important to alleviate the symptoms, but fundamental treatment methods for long-term management of inflammation are also important.

Bronchitis refers to an inflammatory response that occurs in mucous membranes of the trachea and bronchi due to external bacterial or viral infection, or physical/chemical stimuli. Excessive inflammatory responses due to bronchitis cause complications, which may increase the risk of developing tuberculosis, acute pneumonia, bronchiectasis, asthma, etc., and mainly cause bronchial edema and pain, which aggravates pain in patients.

Chronic obstructive pulmonary disease refers to a disease group characterized by poor airflow due to respiratory obstruction without causative pulmonary diseases or heart diseases. Chronic obstructive pulmonary disease is a term used to describe a combination of chronic bronchitis and emphysema, which are clinically difficult to distinguish from each other, with chronic bronchitis being accompanied by a persistent cough and phlegm production, and emphysema being characterized by an abnormal enlargement of the alveoli located at the end of the terminal bronchioles and destruction of alveolar walls. Similar to asthma, chronic obstructive pulmonary disease involves respiratory symptoms such as dyspnea, coughing, and sputum production, which in turn impairs lung function and eventually leads to death.

Laryngitis is caused by inflammation of the larynx itself due to viral or bacterial infection, or spreading of inflammation from surrounding tissues, such as that from pharyngitis and tonsillitis, to the larynx. Laryngitis is one of the common cold symptoms. Laryngitis is caused by infectious diseases collectively referred to as upper respiratory tract infections, and it is difficult to clearly distinguish laryngitis, pharyngitis, and bronchitis from one another.

These respiratory diseases, although there are some differences in their causes and symptoms, have common features as inflammatory diseases. Among currently used medicines, bronchodilators have no effect on inflammation that exacerbates the diseases, but simply alleviate the symptoms. Therefore, their long-term use generates drug resistance, and there is concern about aggravation of the diseases. Further, long-term use of steroids known to be effective for inflammation are problematic due to their serious side effects. In addition, bronchodilators and steroids are often used in combination, and since they are developed as inhaled preparations to avoid the problem of side effects of steroids, inhaled preparations exhibit poor medication adherence, as compared with oral preparations. Accordingly, it is necessary to develop drugs which help to alleviate inflammation and coughing of respiratory diseases with fewer side effects, other than steroids which have many side effects.

*Pelargonium sidoides* is a perennial shrub belonging to the family *Geranium,* and about 80% of *Pelargonium sidoides* is distributed in South Africa. Root extracts of *Pelargonium sidoides* are known to be very effective in treating respiratory diseases. In the early 20^{th} century, the root extract of *Pelargonium sidoides* was used in the United States under the trade name of Umckaloabo™ for the treatment of upper respiratory tract infections such as rhinopharyngitis, tonsillitis, sinusitis, bronchitis, etc. (Kauffer, H.J.(1915) Dental Cosmos 57, 1366). It was also proved that ethanol extracts from roots of *Pelargonium sidoides* are clinically effective in the treatment of respiratory infections and in the field of otolaryngology (Kolodziej et al., Deutsche Apotheker Zeitung 143 (12): 55 - 64 (2003)). A main component of *Pelargonium sidoides* extracts is known to be a polyphenol compound, and as such, the content of *Pelargonium sidoides* extracts is determined by calculating the total phenol content in terms of epicatechin (WO2009/011498).

*Hedera helix* is a plant known as English ivy, and an extract of *Hedera helix* leaf has phlegm discharging activity and is used in the treatment of bronchitis (Korean Patent Publication No. 2004-106201). Its main ingredients are reported to be a saponin called hederacoside C (Demirci et al., Pharmazie 59, 770-774, 2004 and WO2004/087183). In addition to phlegm discharging activity, *Hedera helix* leaf extracts are reported to exhibit antibacterial activity, antifungal activity, antileishmanial activity, digestion promoting activity, etc. (Majester-Savornin, et al., Planta Med. 57, 260-262, 1991; Exp. Parasitol. 116, 340-345, 2007; Mendel et al., J. Ethnopharmacol. 134, 796-802, 2011). Hederacoside C, which is an active ingredient in *Hedera helix* leaves, has also been reported to have many properties such as anti-inflammatory activity, antibacterial activity, antifungal activity, etc. (Gepdiremen et al., Phytomedicicne 12, 440-444, 2005).

Meanwhile, it has been reported (WO 2014/060525) that a composite liquid formulation including both *Pelargonium sidoides* and *Hedera helix* extracts maintains physical stability without precipitation for a long time. However, polyphenols included in *Pelargonium sidoides* and most natural products are easily oxidized by oxygen and transition metal ions in an aqueous solution and converted into quinones, and thus the content of the main component reduces over time (Akagawa et al, Biosci. Biotechnol. Biochen. 67, 2632-2640, 2003). Therefore, it is difficult to prepare a sufficiently effective composite liquid composition including an *Pelargonium sidoides* extract by only securing physical stability. Accordingly, it is necessary to develop a formulation capable of securing storage stability of the polyphenol compound, which is the main component of *Pelargonium sidoides* extracts, in a composite liquid composition.

### DESCRIPTION OF EMBODIMENTS

### TECHNICAL PROBLEM

The object of the present invention is to provide a composite liquid pharmaceutical composition including a combination of a *Pelargonium sidoides* extract and a *Hedera helix* leaf extract, in which storage stability of a main component of *Pelargonium sidoides* is improved.

Another object of the present invention is to provide a method of preparing the composite liquid pharmaceutical composition.

### SOLUTION TO PROBLEM

An aspect of the present invention provides a liquid pharmaceutical composition including a *Hedera helix* leaf extract and a stabilized dry powder of *Pelargonium sidoides,* wherein the stabilized dry powder is a dry powder of a mixture including a *Pelargonium sidoides* extract and maltodextrin.

Another aspect of the present invention provides a method of preparing the liquid pharmaceutical composition according to the present invention, the method including adding and dissolving maltodextrin in the *Pelargonium sidoides* extract to prepare a mixed solution; drying and pulverizing the mixed solution to prepare the stabilized dry powder of *Pelargonium sidoides;* mixing the stabilized dry powder of *Pelargonium sidoides,* the *Hedera helix* leaf extract, and pharmaceutically acceptable additives in water to prepare a mixed solution; and filtering the mixed solution to obtain a filtrate.

### ADVANTAGEOUS EFFECTS OF INVENTION

A composite liquid pharmaceutical composition including a *Hedera helix* leaf extract and a stabilized dry powder including a *Pelargonium sidoides* extract and maltodextrin according to an aspect of the present invention exhibits remarkably high storage stability of a main component of *Pelargonium sidoides* even in a liquid phase and also high storage stability of a main component of the *Hedera helix* leaf extract which is included in combination. Therefore, according to the present invention, it is possible to prepare a composite liquid pharmaceutical composition including the *Pelargonium sidoides* extract and the *Hedera helix* leaf extract, in which storage stabilities of the main components are excellent.

### BEST MODE

Hereinafter, the present invention will be described in more detail.

Unless defined otherwise, all technical terms used herein have the same meaning as commonly understood by one of the ordinary skill in the art to which the present invention belongs. Further, although preferred methods or samples are described in this specification, similar or equivalent ones are also included in the scope of the present invention. Numerical values described herein are also considered to include the meaning of "about" unless explicitly stated otherwise. The contents of all publications referred to herein are incorporated herein by reference in their entirety.

An aspect of the present invention provides a liquid pharmaceutical composition including a *Hedera helix* leaf extract and a stabilized dry powder of *Pelargonium sidoides,* wherein the stabilized powder is a dry powder of a mixture including a *Pelargonium sidoides* extract and maltodextrin.

The Pelargonium sidoides extract may be extracted by using water, an organic solvent, or a combination thereof, and the organic solvent may be C₁₋₃ lower alcohols, acetone, chloroform, methylene chloride, ether, ethyl acetate, hexane, or a combination thereof, but is not limited thereto. In a specific embodiment, the *Pelargonium sidoides* extract is a water or C₁₋₃ lower alcohol extract. The C₁₋₃ alcohol may be exemplified by methanol, ethanol, and propanol, and most preferably, ethanol. In a preferred specific embodiment, the *Pelargonium sidoides* ethanol extract may be about 11% ethanol extract.

In the present specification, it is apparent to those skilled in the art that herbal medicines constituting herbal medicine compositions are considered to belong to the same genus, and the herbal medicine compositions include all herbal medicines (crude drugs) of the same genus, which are effective for the respiratory diseases.

The *Pelargonium sidoides* extract may be prepared by any extraction method known to those skilled in the herbal medicine extraction field, and for example, the *Pelargonium sidoides* extract may be prepared as a liquid extract by performing an extraction method such as hot water extraction, cold extraction, reflux cooling extraction or ultrasonic extraction, preferably, by consecutively performing cold extraction once to five times at an extraction temperature of 10°C to 80°C, preferably, at room temperature (about 25°C) for 10 hours to 80 hours, preferably for 2 days to 3 days. Maltodextrin is added to and dissolved in the solvent extract thus obtained, followed by drying and pulverizing, thereby preparing the stabilized dry powder. The drying and pulverizing may be performed by any drying and pulverizing method, and in a specific embodiment, the stabilized dry powder may be prepared by vacuum drying after filtration under reduced pressure. Specifically, vacuum drying may be performed by using a rotary evaporator at 20°C to 100°C, preferably about 50°C to obtain the dry powder.

The dry powder of the mixture of the *Pelargonium sidoides* extract and maltodextrin (hereinafter, abbreviated to "stabilized dry powder of *Pelargonium sidoides* extract) includes maltodextrin as a stabilizer, thereby improving storage stability of the main component of the *Pelargonium sidoides* extract. In a commercially available syrup formulation of the *Pelargonium sidoides* extract, glycerol is used to improve storage stability of the *Pelargonium sidoides* extract (WO2008-125240). It was confirmed that as compared with use of glycerol, use of maltodextrin remarkably improves storage stability of the main component of the *Pelargonium sidoides* extract (see Experimental Example 1). Particularly, under accelerated conditions, storage stability of the main component of the *Pelargonium sidoides* extract of a liquid formulation of Comparative Example 2 was greatly reduced after 3 months, whereas a content of the main component of the *Pelargonium sidoides* extract in a liquid formulation of Example 2 after 3 months was very similar to an initial content thereof, indicating very high storage stability. Therefore, it was found that the liquid pharmaceutical composition according to the present invention showed very high stability of the main component when it was stored for a long time.

A content of maltodextrin in the stabilized dry powder of *Pelargonium sidoides* may be any content which may increase storage stability of the main component of *Pelargonium sidoides.* In a specific embodiment, the stabilized dry powder of *Pelargonium sidoides* may be a dry powder of a mixture including the *Pelargonium sidoides* extract and maltodextrin at a weight ratio of a dry residue of *Pelargonium* sidoides:maltodextrin of 1:2 to 10.

The "dry residue of *Pelargonium sidoides"* refers to a residue obtained by drying any solvent extract of *Pelargonium sidoides* until a loss on drying becomes about 3.0% or less.

The *Hedera helix* leaf extract may be extracted by using water, an organic solvent, or a combination thereof, and the organic solvent may be C₁₋₃ lower alcohols, acetone, chloroform, methylene chloride, ether, ethyl acetate, hexane, or a combination thereof, but is not limited thereto. The C₁₋₃ alcohol may be exemplified by methanol, ethanol, and propanol, and most preferably, ethanol. In a specific embodiment, the *Hedera helix* leaf ethanol extract may be an about 30% ethanol extract.

In a specific embodiment, the *Hedera helix* leaf extract may be in a form of a dry extract which is a dry powder of a *Hedera helix* leaf solvent extract. The dry extract of the *Hedera helix* leaf extract may be obtained by concentrating the *Hedera helix* leaf solvent extract under reduced pressure, followed by freeze-drying.

The *Hedera helix* leaf extract may be prepared by any extraction method known to those skilled in the herbal medicine extraction field, and for example, the *Hedera helix* leaf extract may be prepared as a liquid solvent extract by performing an extraction method such as hot water extraction, cold extraction, reflux cooling extraction or ultrasonic extraction, preferably, by consecutively performing cold extraction once to five times at an extraction temperature of 10°C to 80°C, preferably, at room temperature (about 25°C) for 10 hours to 80 hours, preferably for 2 days to 3 days. The solvent extract thus obtained is concentrated under reduced pressure, and the filtered extract is concentrated under reduced pressure by using a rotary evaporator at 20 to 100°C, and preferably at room temperature (about 25°C), followed by freeze-drying, thereby preparing the dry extract of the *Hedera helix* leaf extract.

In the liquid pharmaceutical composition, a combination ratio of the stabilized dry powder of *Pelargonium sidoides* and the *Hedera helix* leaf extract is not particularly limited. In a specific embodiment, the liquid pharmaceutical composition may include the stabilized dry powder of *Pelargonium sidoides* and the dry extract of the *Hedera helix* leaf extract at a weight ratio of about 2:1 to about 50:1. In another specific embodiment, the herbal medicine composition may include the stabilized dry powder of *Pelargonium sidoides* and the dry extract of the *Hedera helix* leaf extract at a weight ratio of about 2:1 to about 10:1. According to Korean Patent Publication No. 2016-0081359, it was demonstrated that a composite composition of the *Pelargonium sidoides* extract and the *Hedera helix* leaf extract exhibits synergistic immune enhancement, anti-inflammatory, and antitussive effects, as compared with single use thereof, and therefore, it may be effectively used in prevention, treatment, or improvement of various respiratory diseases. The above patent document is incorporated herein by reference in its entirety.

The liquid pharmaceutical composition may have any formulation known as the liquid pharmaceutical composition, and for example, a liquid formulation, a single-serving drink formulation, a syrup formulation, or a gel-type formulation. Preparation of the various liquid formulations may be appropriately performed by those skilled in the art by using a preparation method known in the art.

The liquid pharmaceutical composition may further include a pharmaceutically acceptable additive selected from the group consisting of pharmaceutically acceptable solvents, diluents, preservatives, sweeteners, flavors, pH adjusters, viscosity modifiers, colorants, and any combinations thereof, depending on specific formulations.

The solvent may serves as a solvent in the liquid pharmaceutical composition, and the solvent may include water, physiological saline, ethanol, a mixture of ethanol and purified water, or isopropyl alcohol, but is not limited thereto. Most preferably, the solvent is water.

The diluent may be any diluent commonly used in the liquid pharmaceutical composition. For example, the diluent may include dextrin, maltodextrin, a sorbitol solution, a mannitol solution, glycerin, liquid fructose, sugar syrup, or any combination, but is not limited thereto.

The preservative may be any preservative commonly used in the liquid pharmaceutical composition. For example, the preservative may include benzoic acid, sodium benzoate, potassium benzoate, calcium benzoate, methyl p-hydroxybenzoate, ethyl p-hydroxybenzoate, propyl p-hydroxybenzoate, isopropyl p-hydroxybenzoate, butyl p-hydroxybenzoate, isobutyl p-hydroxybenzoate, sorbic acid, potassium sorbate, sodium sorbate, dehydroacetic acid, sodium dehydroxyacetate, chlorobutanol, benzalkonium chloride, benzethonium chloride, phenol (p-type), cresol, chlorocresol, benzyl alcohol, or any combination thereof, but is not limited thereto.

The sweetener may be any natural sweetener or synthetic sweetener commonly used in the liquid pharmaceutical composition. The natural sweetener may include saccharides such as white sugar, compressible sugar, confectioners' sugar, glucose, syrup, fructose, isomerized sugar, maltose, honey, lactose, xylose, fructooligosaccharide, etc.; sugar alcohols such as sorbitol, d-sorbitol, maltitol, mannitol, xylitol, erythritol, etc.; glycosides such as glycyrrhizin and salts thereof, stevioside, etc.; and proteins such as thaumatin, etc. The synthetic sweetener may include aspartame; saccharin, saccharin derivatives including saccharin calcium, and saccharin sodium; Alitame; acesulfame-K; sucralose; L-sugar; glycerin; cyclodextrins and derivatives thereof such as beta-cyclodextrin, hydroxypropyl beta-cyclodextrin, gamma-cyclodextrin, alpha-cyclodextrin, etc.; dextrose, lactulose, reduced palatinose, trehalose, isomalt, isomaltooligosaccharide, etc. These natural sweeteners or synthetic sweeteners may be used alone or in combination.

The flavor may be any flavor which is commonly used in the liquid pharmaceutical composition. The flavor may include mint oil, methyl salicylate, nutmeg oil, orange flower oil, orange flower water, orange oil, sweet orange peel tincture, compound orange spirit, peppermint, peppermint oil, peppermint spirit, pine needle oil, rose oil, stronger rose water, spearmint, spearmint oil, thymol, tolu balsam tincture, vanillin, vanilla, vanilla tincture, fennel oil, glycyrrhiza, pure glycyrrhiza extract, liquid glucose, benzaldehyde, anethole, caraway oil, caraway, cardamom oil, almond oil, cardamom seed, cardamom tincture, compound cardamom, cherry juice, cinnamon, cinnamon oil, clove oil, cocoa, coriander oil, eriodictyon fluid extract, eriodictyon, ethyl acetate, ethyl vanillin, eucalyptus, lavender oil, lemon oil, menthol, ginger, various fruit flavors, and other non-toxic flavors, and these flavors may be used alone or in combination of two or more thereof.

The pH adjuster may be any pH adjuster which is commonly used in the liquid pharmaceutical composition. For example, sodium hydroxide, potassium hydroxide, prolamine, ammonia water, ammonium carbonate, etc. may be used as an alkalizer, and non-toxic pH adjusters such as acetic acid, citric acid, malic acid, tartaric acid, various phosphates including hydrochloric acid may be used as an acidifier.

The viscosity modifier may be any viscosity modifier which is commonly used in the liquid pharmaceutical composition. For example, the viscosity modifier may be selected from xanthan gum, acacia gum, tragacanth powder, polyvinylpyrrolidone, ethyl cellulose, carboxymethylcellulose sodium, and combinations thereof.

The colorant may be any colorant which is commonly used in the liquid pharmaceutical composition. For example, the colorant may include various pharmaceutically acceptable colors such as red #3, red #40, yellow #4, yellow #5, yellow #203, green #3, blue #1, and blue #2 which are tar colors for internal use, and other non-toxic colors, and they may be used alone or in combination of two or more thereof.

In a specific embodiment, the liquid pharmaceutical composition may be an oral liquid formulation prepared by dissolving maltodextrin, sorbitol, potassium sorbate, xylitol, sucralose, and citric acid which are pharmaceutical additives, together with the stabilized dry powder of *Pelargonium sidoides* and the *Hedera helix* leaf extract which are active ingredients, in water (particularly, purified water).

Results of performing a storage stability test for the liquid pharmaceutical composition of the present invention showed that a liquid formulation (Example 2) including the stabilized dry powder of *Pelargonium sidoides* including maltodextrin as a stabilizer exhibited remarkably excellent storage stability of the main components, as compared with a liquid formulation (Comparative Example 2) including a liquid mixture of glycerol as a stabilizer and the *Pelargonium sidoides* extract. Specifically, results of stability tests under long-term storage conditions (25°C/60% relative humidity) and accelerated conditions (40°C/75% relative humidity) showed that under both conditions, the liquid formulation of Example 2 had a much high content of a phenolic compound which is an index component of *Pelargonium sidoides* over time, as compared with the liquid formulation of Comparative Example 2, indicating that the phenolic compound in the liquid formulation of Example 2 was hardly degraded. Accordingly, it was confirmed that the liquid pharmaceutical composition including the stabilized dry powder of *Pelargonium sidoides* and the *Hedera helix* leaf extract according to the present invention may be prepared as a composite liquid formulation having very excellent storage stability of the active ingredient (see Experimental Example 1).

The respiratory diseases may be any respiratory diseases which are known to be treated, improved, and/or prevented by the immune enhancement, anti-inflammatory, and antitussive effects. For example, the respiratory diseases may include respiratory infectious diseases, asthma, chronic bronchitis, and chronic obstructive pulmonary disease, and the respiratory infectious diseases may include cold, bronchitis, sinusitis, tonsillitis, and sore throat, but are not limited thereto.

Furthermore, since the liquid pharmaceutical composition according to an aspect of the present invention includes herbal medicines which have been used for a very long time and proved to have safety, it has no toxicity and side effects, and therefore, the liquid pharmaceutical composition may be used with safety when it is taken for a long time in order to prevent or treat respiratory diseases.

The liquid pharmaceutical composition may be administered to many different kinds of mammals including rats, mice, livestock, humans, etc. via various routes of administration, preferably, via an oral administration.

In the liquid pharmaceutical composition, a mixing ratio of the herbal medicine components may be appropriately increased or decreased within a range for effective treatment or prevention of respiratory diseases, and the herbal medicine components constituting the composition according to the present invention may be included in an amount of 0.01% by weight to 80% by weight, more specifically, 1% by weight to 50% by weight.

In order to obtain the effect of preventing or treating respiratory diseases, the active ingredients of the liquid pharmaceutical composition may be administered at a dose of 0.01 g/kg/day to 10 g/kg/day, preferably, 0.1 g/kg/day to 5 g/kg/day once a day or several times a day, based on the dry powder of the solvent extract, and the dose may be appropriately increased or decreased depending on a patient's age, sex, body weight, diet, excretion rate, severity, or other drugs currently taken. Therefore, the liquid pharmaceutical composition should be prepared taking into account the range of effective doses, and unit dosage forms thus formulated may be administered with specialized dosing regimens or several times at regular intervals according to the judgment of a practitioner who monitors or observes administration of the drug and individual's needs, as needed.

Another aspect of the present invention provides a method of preparing the liquid pharmaceutical composition according to an aspect of the present invention, the method including adding and dissolving maltodextrin in the *Pelargonium sidoides* extract to prepare a mixed solution; drying and pulverizing the mixed solution to prepare the stabilized dry powder of *Pelargonium sidoides;* mixing the stabilized dry powder of *Pelargonium sidoides,* the *Hedera helix* leaf extract, and pharmaceutically acceptable additives in water to prepare a mixed solution; and filtering the mixed solution to obtain a filtrate.

The detailed description of the liquid pharmaceutical composition according to an aspect of the present invention may be applied as it is to description of the method of preparing the liquid pharmaceutical composition according to the present aspect.

In a specific embodiment, the pharmaceutical additives are first dissolved in a solvent, and then in this solution, the stabilized dry powder of *Pelargonium sidoides* and the *Hedera helix* leaf extract are mixed and filtered to obtain a filtrate. In a specific embodiment, the pharmaceutical additives include maltodextrin, a sorbitol solution, xylitol, potassium sorbate, sucralose, and citric acid, and the solvent is purified water.

The filtrate may be used directly as the liquid formulation or may be prepared as a liquid pharmaceutical composition of a different formulation according to a common formulation method known in the art.

### MODE OF INVENTION

Hereinafter, the present invention will be described in detail with reference to the following Examples. However, these Examples are for illustrative purposes only, and the scope of the present invention is not intended to be limited by these Examples.

### Example 1: Preparation of stabilized dry powder of Pelargonium sidoides

A stabilized dry powder of *Pelargonium sidoides* was prepared as a mixture of 11% ethanol extract of *Pelargonium sidoides* and maltodextrin.

First, roots of *Pelargonium sidoides* were washed to remove contaminants, dried well and then used in preparation of the extract. 2 kg of 35% by weight of an ethanol aqueous solution was added to 1 kg of roots of *Pelargonium sidoides* which had been well pulverized by a pulverizer, and left at room temperature for 20 hours. Thereafter, the mixture was extracted with 8 kg of 6% by weight of an ethanol aqueous solution for 10 hours, followed by filtration. A dry residue of the filtrate was 1.78% by weight.

0.41 kg of maltodextrin was dissolved in 5 kg of the liquid extract, and this solution was dried under vacuum at 50°C to obtain the stabilized dry powder of *Pelargonium sidoides.*

### Comparative Example 1: Preparation of liquid mixture of Pelargonium sidoides and glycerol

A liquid mixture of 11% ethanol extract of *Pelargonium sidoides* and glycerol was prepared.

First, roots of *Pelargonium sidoides* were washed to remove contaminants, dried well and then used in preparation of the extract. 2 kg of 35% by weight of an ethanol aqueous solution was added to 1 kg of roots of *Pelargonium sidoides* which had been well pulverized by a pulverizer, and left at room temperature for 20 hours. Thereafter, the mixture was extracted with 8 kg of 6% by weight of an ethanol aqueous solution for 10 hours, followed by filtration. A dry residue of the filtrate was 1.78% by weight.

1 kg of glycerol was mixed with 4 kg of the liquid extract to obtain the liquid mixture of *Pelargonium sidoides* and glycerol.

### Example 2: Preparation of liquid formulation including stabilized dry powder of Pelargonium sidoides and Hedera helix leaf extract

A liquid formulation was prepared according to a composition of the following Table 1. Maltodextrin, a sorbitol solution, xylitol, potassium sorbate, xylitol, sucralose, and citric acid described in the following Table 1 were dissolved in purified water, and then the stabilized dry powder of *Pelargonium sidoides* (mixed dry powder of 11% ethanol extract and maltodextrin) prepared in Example 1 and 30% ethanol dry extract of *Hedera helix* leaf were added and dissolved, and sufficiently mixed and filtered to prepare the liquid formulation.

**[Table 1**

| Section | Components | Example 2 |
|---|---|---|
| | | g/100 mL |
| Main component | Mixed dry powder of 11% ethanol extract of *Pelargonium* sidoides/maltodextrin | 1.144 |
| Main component | 30% ethanol dry extract of *Hedera helix* leaf | 0.389 |
| Diluent | Maltodextrin | 0.432 |
| | Sorbitol solution | 15.000 |
| Preservative | Potassium sorbate | 0.117 |
| Sweetener | Xylitol | 7.000 |
| | Sucralose | 0 . 020 |
| pH adjuster | Citric acid | 0.040 |
| Solvent | Purified water | Proper amount |

### Comparative Example 2: Preparation of composite liquid formulation including liquid mixture of Pelargonium sidoides and Hedera helix leaf extract

A liquid formulation was prepared according to a composition of the following Table 2. A sorbitol solution, xylitol, potassium sorbate, sucralose, and citric acid, as in a composition of Comparative Example 1 described in the following Table 2, were dissolved in purified water, and then the liquid mixture of *Pelargonium sidoides* and glycerol (liquid mixture of 11% ethanol extract of *Pelargonium sidoides* and glycerol) prepared in Comparative Example 1 and 30% ethanol dry extract of *Hedera helix* leaf were added and dissolved, and sufficiently mixed and filtered to prepare the liquid formulation.

**[Table 2]**

| Section | Components | Comparative Example 2 |
|---|---|---|
| | | g/100 mL |
| Main component | Liquid mixture of 11% ethanol extract of *Pelargonium* sidoides/glycerin | 17.160 |
| Main component | 30% ethanol dry extract of *Hedera helix* leaf | 0.389 |
| Diluent | Maltodextrin | 0.432 |
| | Sorbitol solution | 15.000 |
| Preservative | Potassium sorbate | 0.117 |
| Sweetener | Xylitol | 7.000 |
| | Sucralose | 0.020 |
| pH adjuster | Citric acid | 0.040 |
| Solvent | Purified water | Proper amount |

### Experimental Example 1: Stability test of composite liquid formulation including Pelargonium sidoides and Hedera helix leaf extracts

The composite liquid formulations including *Pelargonium sidoides* and *Hedera helix* leaf extracts of Example 2 and Comparative Example 2 were stored for 1 month to 3 months under long-term storage conditions of 25°C/60% relative humidity and under accelerated conditions of 40°C/75% relative humidity, and then contents of total phenolic compounds which are the index components of *Pelargonium sidoides* and hederacoside C which is the index component of Hedera helix leaf, which remained in each composite liquid formulation, were measured. Results are shown in the following Table 3 (unit: %).

**[Table 3]**

| | | Initial | | 1 month | | 3 months | |
|---|---|---|---|---|---|---|---|
| | | Total phenols | Hederacoside C | Total phenols | Hederacoside C | Total phenols | Hederacoside C |
| Long-term storage conditions | Example 2 | 100 | 100 | 100 | 101.0 | 103 | 103 |
| | Comparative Example 2 | 100 | 100 | 98.7 | 96.5 | 84.0 | 100 |
| Accelerated conditions | Example 2 | 100 | 100 | 98.0 | 100 | 92.0 | 102 |
| | Comparative Example 2 | 100 | 100 | 92.8 | 95.8 | 66.0 | 97 |

While the present invention has been particularly shown and described with reference to preferred embodiments, it will be understood by those skilled in the art that various changes in form and detail may be made therein without departing from essential characteristics of the present invention. Therefore, it should be understood that embodiments described herein should be considered in a descriptive sense only and not for purposes of limitation. The scope of the present invention is defined by the appended claims rather than by the foregoing description.

## Claims

1. A liquid pharmaceutical composition comprising a *Hedera helix* leaf extract and a stabilized dry powder of *Pelargonium sidoides,* wherein the stabilized dry powder is a dry powder of a mixture comprising a *Pelargonium sidoides* extract and maltodextrin.

2. The liquid pharmaceutical composition of claim 1, wherein the *Pelargonium sidoides* extract is a water or C₁₋₃ lower alcohol extract.

3. The liquid pharmaceutical composition of claim 1, wherein in the mixture comprising a *Pelargonium sidoides* extract and maltodextrin, a dry residue of a *Pelargonium sidoides* extract and maltodextrin are mixed at a weight ratio of 1:2 to 10.

4. The liquid pharmaceutical composition of claim 1, wherein the *Hedera helix* leaf extract is a water or C₁₋₃ lower alcohol extract.

5. The liquid pharmaceutical composition of claim 4, wherein the *Hedera helix* leaf extract is a dry *Hedera helix* leaf extract, which is a dry powder of the water or C₁₋₃ lower alcohol extract.

6. The liquid pharmaceutical composition of claim 5, wherein the liquid pharmaceutical composition comprises the stabilized dry powder of *Pelargonium sidoides* and the dry *Hedera helix* leaf extract at a weight ratio of 2:1 to 50:1.

7. The liquid pharmaceutical composition of claim 1, wherein the liquid pharmaceutical composition has a liquid formulation, a single-serving drink formulation, a syrup formulation, or a gel-type formulation.

8. The liquid pharmaceutical composition of claim 1, wherein the liquid pharmaceutical composition further comprises pharmaceutical additives selected from the group consisting of diluents, preservatives, sweeteners, pH adjusters, and any combination thereof.

9. The liquid pharmaceutical composition of claim 8, wherein the liquid pharmaceutical composition comprises maltodextrin, sorbitol, potassium sorbate, xylitol, sucralose, and citric acid.

10. The liquid pharmaceutical composition of claim 1, wherein the liquid pharmaceutical composition is used for treatment of respiratory tract infection.

11. The liquid pharmaceutical composition of claim 10, wherein the respiratory tract infection is cold, bronchitis, sinusitis, tonsillitis, or sore throat.

12. A method of preparing the liquid pharmaceutical composition of any one of claims 1 to 11, the method comprising:
adding and dissolving maltodextrin in a *Pelargonium sidoides* extract to prepare a mixed solution;
drying and pulverizing the mixed solution to prepare a stabilized dry powder of *Pelargonium sidoides;*
mixing the stabilized dry powder of *Pelargonium sidoides,* a *Hedera helix* leaf extract, and pharmaceutically acceptable additives in water to prepare a mixed solution; and
filtering the mixed solution to obtain a filtrate.

13. The method of claim 12, wherein the drying and pulverizing are performed by vacuum drying.

## Patentansprüche

1. Flüssige pharmazeutische Zusammensetzung, die einen *Hedera-helix-*Blattextrakt und ein stabilisiertes trockenes Pulver von *Pelargonium sidoides* umfasst, wobei das stabilisierte trockene Pulver ein trockenes Pulver eines Gemischs, das einen *Pelargonium-sidoides-Extrakt* und Maltodextrin umfasst, ist.

2. Flüssige pharmazeutische Zusammensetzung gemäß Anspruch 1, wobei der *Pelargonium-sidoides-Extrakt* ein wässriger Extrakt oder ein Extrakt in einem niederen C₁₋₃- Alkohol ist.

3. Flüssige pharmazeutische Zusammensetzung gemäß Anspruch 1, wobei bei dem Gemisch, das einen *Pelargonium-sidoides-Extrakt* und Maltodextrin umfasst, ein trockener Rückstand eines *Pelargonium-sidoides-*Extrakts und Maltodextrin in einem Gewichtsverhältnis von 1:2 bis 10 gemischt werden.

4. Flüssige pharmazeutische Zusammensetzung gemäß Anspruch 1, wobei der Hedera-helix-Blattextrakt ein wässriger Extrakt oder ein Extrakt in einem niederen C₁₋₃- Alkohol ist.

5. Flüssige pharmazeutische Zusammensetzung gemäß Anspruch 4, wobei der Hedera-helix-Blattextrakt ein trockener Hedera-helix-Blattextrakt ist, bei dem es sich um ein trockenes Pulver des wässrigen Extrakts oder des Extrakts in einem niederen C₁₋₃- Alkohol handelt.

6. Flüssige pharmazeutische Zusammensetzung gemäß Anspruch 5, wobei die flüssige pharmazeutische Zusammensetzung das stabilisierte trockene Pulver von *Pelargonium sidoides* und den trockenen *Hedera-helix-Blatt*extrakt in einem Gewichtsverhältnis von 2:1 bis 50:1 umfasst.

7. Flüssige pharmazeutische Zusammensetzung gemäß Anspruch 1, wobei die flüssige pharmazeutische Zusammensetzung eine flüssige Zubereitung, eine Getränkzubereitung als einzelne Portion, eine Sirupzubereitung oder eine Zubereitung des Geltyps aufweist.

8. Flüssige pharmazeutische Zusammensetzung gemäß Anspruch 1, wobei die flüssige pharmazeutische Zusammensetzung weiterhin pharmazeutische Additive umfasst, die aus der Gruppe ausgewählt sind, die aus Verdünnungsmitteln, Konservierungsmitteln, Süßungsmitteln, pH-Regulatoren und einer beliebigen Kombination davon besteht.

9. Flüssige pharmazeutische Zusammensetzung gemäß Anspruch 8, wobei die flüssige pharmazeutische Zusammensetzung Maltodextrin, Sorbit, Kaliumsorbat, Xylit, Sucralose und Zitronensäure umfasst.

10. Flüssige pharmazeutische Zusammensetzung gemäß Anspruch 1, wobei die flüssige pharmazeutische Zusammensetzung für die Behandlung einer Atemwegsinfektion verwendet wird.

11. Flüssige pharmazeutische Zusammensetzung gemäß Anspruch 10, wobei es sich bei der Atemwegsinfektion um Erkältung, Bronchitis, Sinusitis, Tonsillitis oder Halsschmerzen handelt.

12. Verfahren zur Herstellung der flüssigen pharmazeutischen Zusammensetzung gemäß einem der Ansprüche 1 bis 11, wobei das Verfahren umfasst:
Hinzufügen von Maltodextrin zu einem *Pelargonium-sidoides-Extrakt* und Auflösen in demselben zur Herstellung einer gemischten Lösung;
Trocknen und Pulverisieren der gemischten Lösung zur Herstellung eines stabilisierten trockenen Pulvers von *Pelargonium sidoides;*
Mischen des stabilisierten trockenen Pulvers von *Pelargonium sidoides,* eines Hedera-helix-Blattextrakts und von pharmazeutisch annehmbaren Additiven in Wasser zur Herstellung einer gemischten Lösung; und
Filtrieren der gemischten Lösung, wobei man ein Filtrat erhält.

13. Verfahren gemäß Anspruch 12, wobei das Trocknen und Pulverisieren durch Vakuumtrocknen durchgeführt wird.

## Revendications

1. Composition pharmaceutique liquide comprenant un extrait de feuille de *Hedera hélix* et une poudre sèche stabilisée de *Pelargonium sidoides,* dans laquelle la poudre sèche stabilisée est une poudre sèche d'un mélange comprenant un extrait de *Pelargonium sidoides* et de la maltodextrine.

2. Composition pharmaceutique liquide selon la revendication 1, dans laquelle l'extrait de *Pelargonium sidoides* est un extrait dans l'eau ou un alcool inférieur en C₁ à ₃.

3. Composition pharmaceutique liquide selon la revendication 1, dans laquelle dans le mélange comprenant un extrait de *Pelargonium sidoides* et de la maltodextrine, un résidu sec d'un extrait de *Pelargonium sidoides* et de la maltodextrine sont mélangés à un rapport en poids de 1:2 à 10.

4. Composition pharmaceutique liquide selon la revendication 1, dans laquelle l'extrait de feuille de *Hedera hélix* est un extrait dans l'eau ou un alcool inférieur en C₁ à ₃.

5. Composition pharmaceutique liquide selon la revendication 4, dans laquelle l'extrait de feuille de *Hedera hélix* est un extrait de feuille de *Hedera hélix* sec, qui est une poudre sèche de l'extrait dans l'eau ou un alcool inférieur en C_{1 à 3}.

6. Composition pharmaceutique liquide selon la revendication 5, dans laquelle la composition pharmaceutique liquide comprend la poudre sèche stabilisée de *Pelargonium sidoides* et l'extrait de feuille de *Hedera hélix* sec à un rapport en poids de 2:1 à 50:1.

7. Composition pharmaceutique liquide selon la revendication 1, dans laquelle la composition pharmaceutique liquide a une formulation liquide, une formulation de boisson en portion individuelle, une formulation de sirop, ou une formulation de type gel.

8. Composition pharmaceutique liquide selon la revendication 1, dans laquelle la composition pharmaceutique liquide comprend en outre des additifs pharmaceutiques choisis dans le groupe constitué par des diluants, des conservateurs, des édulcorants, des agents d'ajustement du pH, et toute combinaison de ceux-ci.

9. Composition pharmaceutique liquide selon la revendication 8, dans laquelle la composition pharmaceutique liquide comprend de la maltodextrine, du sorbitol, du sorbate de potassium, du xylitol, du sucralose, et de l'acide citrique.

10. Composition pharmaceutique liquide selon la revendication 1, dans laquelle la composition pharmaceutique liquide est utilisée pour le traitement d'une infection des voies respiratoires.

11. Composition pharmaceutique liquide selon la revendication 10, dans laquelle l'infection des voies respiratoires est un rhume, une bronchite, une sinusite, une amygdalite, ou un mal de gorge.

12. Procédé de préparation de la composition pharmaceutique liquide de l'une quelconque des revendications 1 à 11, le procédé comprenant :
l'ajout et la dissolution de maltodextrine dans un extrait de *Pelargonium sidoides* pour préparer une solution mixte ;
le séchage et la pulvérisation de la solution mixte pour préparer une poudre sèche stabilisée de *Pelargonium sidoides ;*
le mélange de la poudre sèche stabilisée de *Pelargonium sidoides,* d'un extrait de feuille de *Hedera helix,* et d'additifs pharmaceutiquement acceptables dans de l'eau pour préparer une solution mixte ; et
la filtration de la solution mixte pour obtenir un filtrat.

13. Procédé selon la revendication 12, dans lequel le séchage et la pulvérisation sont réalisés par séchage sous vide.
